# EUROPEAN PATENT SPECIFICATION

(11) **EP 0 444 775 B1**
(45) Date of publication and mention of the grant of the patent: **19.06.1996**
(21) Application number: 91300540.1
(22) Date of filing: 24.01.1991
(51) Int. Cl.: C12N 15/61, C12N 9/90, C07H 21/04

(54) **Recombinant DNA expression vectors and DNA compounds that encode isopenicillin N epimerase activity**
Rekombinante DNA-Expressionsvektoren und DNA-Zusammensetzungen, die Isopenizillin N-Epimerase-Aktivität kodieren
Vecteurs d'expression d'ADN recombinant et composés d'ADN encodant l'activité d'isopénicilline N épimérase

(30) Priority: 26.01.1990 US 470476
(43) Date of publication of application: 04.09.1991
(73) Proprietor: ELI LILLY AND COMPANY, Indianapolis, Indiana 46285 (US)
(72) Inventor: Weigel, Barbara Jean, Indianapolis,Indiana 46208 (US)
(74) Representative: Hudson, Christopher Mark

(56) References cited:
- EP-A- 0 295 724
- EP-A- 0 377 295

## Description

The sulfur-containing [β-lactam antibiotics are the most important clinically, and the isolation of novel lactam compounds continues nearly six decades since the discovery of the penicillins by Fleming. The common structural feature of the penicillins and cephalosporins (including cephamycins) is the [β-lactam ring

These antibiotics are produced by a variety of prokaryotes and lower eukaryotes. The penicillins, exemplified by the compounds penicillin G (benzylpenicillin) or penicillin V, are produced by filamentous fungi, most notably Penicillium chrysogenum. The cephalosporins, first isolated as a product from the lower eukaryote, Cephalosporium acremonium (syn. Acremonium chrysoqenum), are also metabolites of many prokaryotes, especially Streptomyces clavuligerus, S. lipmanii and S. cattleya that also produce cephamycins and other β-lactams such as oxypenams (clavulanic acid) and carbapenems (thienamycin).

The development of cell-free systems from β-lactam-producing organisms has allowed the establishment of the biosynthetic steps in the pathway of the sulfurcontaining β-lactams (penicillins and cephalosporins). The initial steps in the formation of penicillins in filamentous fungi (e.g., P. chrvsogenum), and the cephalosporins produced by both prokaryotes and lower eukaryotes, are identical. ACV synthetase catalyzes the condensation of the amino acid precursors L-a-aminoadipate, L-cysteine, and L-valine to the tripeptide LLD-ACV The next step forms the first β-lactam in the pathway by the cyclization of the tripeptide yielding isopenicillin N (IPN), the precursor to all penicillins, cephalosporins and cephamycins.

After synthesis of lPN, the pathways to cephalosporins and penicillins diverge In Penicillium chrvsoqenum, for example, the a-aminoadipyl side chain of IPN can be exchanged for one of many (nearly 100 to date) hydrophobic side chains derived from the corresponding acyl CoA. One of the most familiar examples is the formation of penicillin G (benzylpenicillin) from phenylacetyl CoA and IPN. However, in the fungus C. acremonium, the a-aminoadipyl side chain is isomerized to produce penicillin N. The five-membered thiazolidine ring of the penicillin is then "expanded" to the six-membered dihydrothiazine ring that is characteristic of the cephalosporins. This reaction is catalyzed by deac- etoxycephalosporin C synthetase (DAOCS) and produces the first cephalosporin in the pathway deacetoxycepha- losporin C (DAOC).

Two goals of beta-lactam antibiotic research are to increase production of known antibiotics and to generate novel useful antibiotics. The cloning of genes encoding enzymes in this pathway provides basic tools useful in achieving these goals. The present invention provides DNA sequences encoding the enzyme isopenicillin N epimerase.

The present invention comprises DNA sequences that encode isopenicillin N epimerase (epimerase) activity from Streptomyces lipmanii. The newly isolated DNA sequence provided herein can be used to produce epimerase activity in large amounts in recombinant host cells. Isopenicillin N epimerase catalyzes the reaction in which penicillin N is formed from isopenicillin N The enzyme converts the L-alpha-aminoadipyl side chain of isopenicillin N to a D-alpha-aminoadipyl side chain. The enzyme is highly unstable, but the reaction catalyzed is a critical step in the biosynthesis of important antibiotics, such as cephalosporins from Cephalosporium acremonium and 7a-methoxycephalosporins or cephamycins from Streptomyces lipmanii and S. clavuliqerus. Thus, it is quite important to be able to produce the activity in large quantities.

The DNA compounds of the present invention encode the epimerase activity in a single open reading frame. Transcription of this open reading frame, followed by translation of the resulting mRNA, yields a single polypeptide chain that possesses epimerase activity. The DNA compound that encodes the epimerase activity was isolated from Streptomyces lipmanii genomic DNA and used to construct recombinant DNA expression vectors. Four types of these expression vectors are especially useful: the first drives high-level expression of the epimerase activity in E. coli; the second in Cephalosporium; the third in Penicillium; and the fourth in Streptomyces.

The DNA compounds encoding the epimerase activity are readily modified to construct expression vectors that increase the efficiency and yield of fermentations involving other organisms, such as Paecilomyces and Streptomyces. Although the epimerase-activity-encoding DNA of the present invention was isolated from S. lipmanii, this DNA can be used to construct vectors that drive expression of epimerase activity in a wide variety of host cells, as illustrated by the E. coli expression vectors described herein. The construction protocols utilized for the E. coli, Cephalosporium, and Penicillium vectors of the invention can be followed to construct analogous vectors for other organisms, merely by substituting, if necessary, the appropriate regulatory elements. The epimerase-encoding DNA compounds of the present invention can be used to construct expression vectors useful for improving the efficiency and yield of fermentations involving a wide variety of penicillin and cephalosporin antibiotic-producing organisms.

The invention provided herein includes DNA compounds that comprise a DNA sequence encoding epimerase activity of Streptomyces lipmanii. One such sequence is comprised by the ~3.5 kb EcoRi restriction fragment of plasmid mOG0292. Also claimed are recombinant DNA vectors, including expression vectors, which are useful in host organisms such as E. coli, Aspergillus, Cephalosporium, Penicillium, and Streptomyces. Specific vectors provided by this invention are the plasmids mOG0292, mOG0297, and pOG0299. The vectors are useful in a method for constructing a recombinant host cell capable of expressing epimerase activity in a recombinant host cell, said method comprising: transforming said host cell with a recombinant DNA expression vector that comprises:
(a) a promoter and translational activating sequence that function in said host cell; and
(b) a DNA sequence encoding epimerase activity positioned for expression from said promoter and translational activating sequence

Another useful method results in the expression of epimerase activity in a recombinant host cell via the culture of microorganisms transformed with an expression vector of the present invention.

The following section provides a more detailed description of the present invention. For purposes of clarity and as an aid in the understanding of the invention, as disclosed and claimed herein, the following items are defined below
amdS - an acetamidase gene; also used in the Figures to denote the Asperqillus nidulans acetamidase gene.
amdSp - the promoter of the amdS gene.
AmR - the apramycin resistance-conferring gene; also used to denote the apramycin-resistant phenotype.

Antibiotic - a substance produced by a microorganism that, either naturally or with limited modification, will inhibit the growth of or kill another microorganism or eukaryotic cell.

Antibiotic Biosynthetic Gene - a DNA segment that encodes an activity necessary for an enzymatic reaction in the process of converting primary metabolites into antibiotics or converting one antibiotic compound into a different antibiotic compound.

Antibiotic-Producing Organism - any organism, including, but not limited to, Streptomyces, Bacillus. Flavobacte- rium, Monospora, Cephalosporium, Paecilomyces, Podospora, Penicillium, and Nocardia, that either produces an antibiotic or contains genes that, if expressed, would produce an antibiotic.

Antibiotic Resistance-Conferring Gene - a DNA segment that encodes an activity that confers resistance to an antibiotic.
ApR - the ampicillin resistance-conferring gene; also used to denote the ampicillin-resistant phenotype
bp - a base pair of double-stranded DNA.
CAT - the chloramphenicol resistance-conferring gene, which encodes an acetyltransferase.
c1857 - a gene encoding a temperature sensitive repressor of the ),pL promoter
clPS - isopenicillin N synthetase or isopenicillin N synthetase-encoding DNA from Cephalosporium acremonium.
Cloning - the process of incorporating a segment of DNA into a recombinant DNA cloning vector.
Coding Sequence - the sequence of DNA in a gene that encodes either the amino acid residue sequence of the protein expressed by the gene or, in the case of rRNA or tRNA genes, the RNA sequence of the rRNA or tRNA expressed by the gene.
Cosmid - a recombinant DNA cloning vector that can replicate in a host cell in the same manner as a plasmid but that can also utilize phage packaging mechanisms.
Epimerase Activity - an enzymatic activity which catalyzes the conversion of isopenicillin N to penicillin N and vice-versa and is encoded by the gene of the present invention or by DNA which can be identified by using the gene of the present invention as a probe.
Gene - a segment of DNA that comprises a promoter, translational activating sequence, coding sequence, and 3' regulatory sequences positioned to drive expression of the gene product, either a protein (and thus necessarily an mRNA), tRNA, or rRNA.
Genomic Library - a set of recombinant DNA cloning vectors into which segments of DNA, which substantially represent the entire genome of a particular organism, have been cloned.
Hybridization - the process of annealing two single-stranded DNA and/or RNA molecules toform a double-stranded molecule that may or may not be completely base-paired.
IPS or IPNS - isopenicillin N synthetase.
Isopenicillin N - has the structure depicted below: 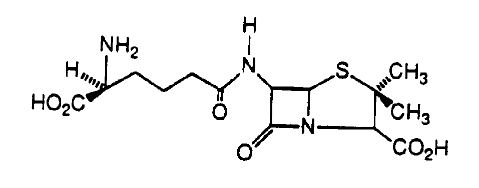
Isopenicillin N Synthetase - an enzyme, also known as cyclase, that catalyzes the formation of isopenicillin N from δ-(L-α-aminoadipyl)-L-cysteinyl-D-valine
Isolated DNA compound - Any DNA sequence, however constructed or synthesized, which is locationally distinct from the genomic DNA of the organism in whcih the sequence occurs.
KmR - the kanamycin resistance-conferring gene also used to denote the kanamycin-resistant phenotype.
lacl - the E. coli lac gene.
lacZa - the promoter and β-galactosidase (LacZ) a-fragment derived from the E. coli lac operon.
M13 ORI - the origin of replication of phage M13.
MCS - a multiple-cloning site.
mRNA - messenger ribonucleic acid.
ORI - a plasmid or vector origin of replication, the DNA sequence that serves as an attachment or start site for DNA polymerase.
Pen DNA - DNA from Penicillium chrysogenum.
Penicillin N - has the structure depicted below: 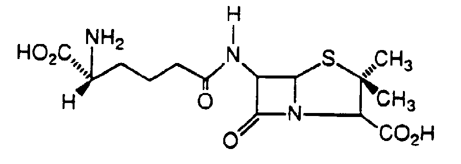
pIPS - the IPS gene or IPS coding sequence of Penicillium chrysogenum.
pIPSp - the promoter of the IPS gene of Penicillium chrysogenum.
pIPSt - the transcription terminator of the IPS gene of Penicillium chrysogenum.
pL - the leftward promoter from bacteriophage lambda.
Promoter - a DNA sequence that directs or initiates the transcription of DNA.
Recombinant DNA Cloning Vector - any autonomously replicating or integrating agent, including, but not limited to, plasmids, comprising a DNA molecule to which one or more additional DNA molecules can be or have been added.
Recombinant DNA Expression Vector - any autonomously replicating or integrating agent, including, but not limited to, plasmids, comprising a promoter and other regulatory sequences positioned to drive expression of a DNA segment that encodes a polypeptide or RNA.
Recombinant DNA sequence - any DNA sequence, excluding the host chromosome from which the DNA sequence is derived, which comprises a DNA sequence which has been isolated, synthesized, or partially synthesized.
Recombinant DNA Vector - any recombinant DNA cloning or expression vector.
Restriction Fragment - any linear DNA molecule generated by the action of one or more enzymes.
rRNA - ribosomal ribonucleic acid.
Sensitive Host Cell - a host cell that cannot grow or whose growth is inhibited in the presence of a given antibiotic without a DNA segment that confers resistance thereto.
TcR - the tetracycline resistance-conferring gene; also used to denote the tetracycline-resistant phenotype.
Transcription Terminator - a DNA sequence that signals the termination of transcription of DNA by RNA polymerase.
Transfectant - a recipient host cell that has undergone transformation by phage DNA or by DNA packaged into a phage particle.
Transformant - a recipient host cell that has undergone transformation.
Transformation - the introduction of DNA into a recipient host cell that changes the genotype and results in a change in the recipient cell.
Translational activating sequence - a regulatory DNA sequence that, when transcribed into mRNA, promotes translation of mRNA into protein.
tRNA - transfer ribonucleic acid.
trp - the promoter and translational activating sequence of the tryptophan operon of E. coli.

### Brief Description of the Figures

The restriction site and function maps presented in the accompanying drawings are approximate representations of the recombinant DNA vectors discussed herein. The restriction site information is not exhaustive; therefore, there may be more restriction sites of a given type on the vector than actually shown on the map.
Figure 1. A restriction site and function map of plasmid mOG0292.
Figure 2. A restriction site and function map of plasmid pCZR111.
Figure 3. A restriction site and function map of plasmid pOG0299.

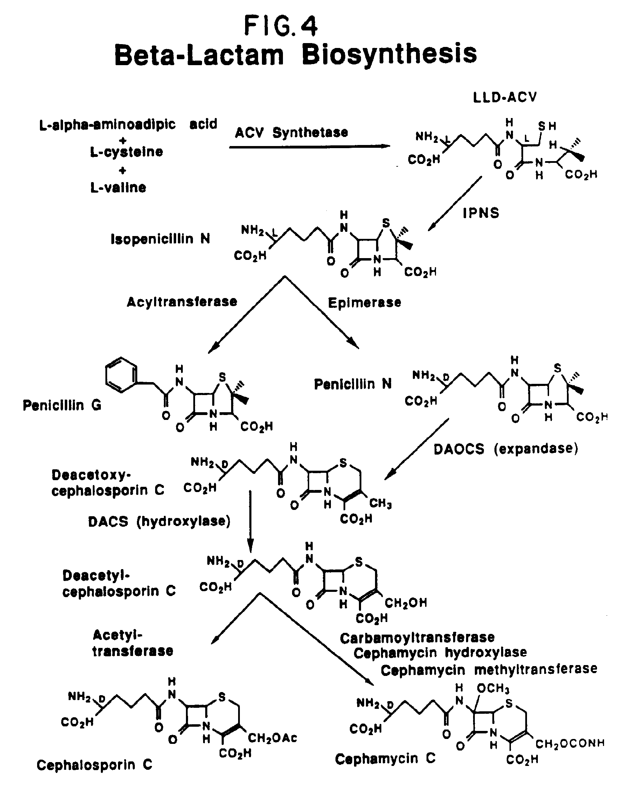

Figure 4. A diagram of beta-lactam biosynthetic pathways.
Figure 5. A restriction site and function map of plasmid pMLC12.
Figure 6. A restriction site and function map of plasmid pLC2.
Figure 7. A restriction site and function map of plasmid p3SR2.
Figure 8. A restriction site and function map of plasmid pUT715.

### Detailed Description of the Invention

The present invention comprises isolated DNA compounds and recombinant DNA cloning and expression vectors that encode the epimerase activity of Streptomyces lipmanii. The sequence of the S. lipmanii epimerase-encoding DNA is depicted below. In the depiction, only the coding strand of the double-stranded DNA molecule is shown, and the DNA is depicted from left to right in the 5' → 3' orientation. The nucleotide sequence is numbered; the numbers appear to the left of the DNA sequence. The amino acid residue sequence of the epimerase encoded by the DNA is listed, following the DNA sequence, from left to right in the amino-terminus - carboxy-terminus direction. 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
 
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 wherein A is a deoxyadenyl residue, G is a deoxyguanyl residue, C is a deoxycytidyl residue, and T is a thymidyl residue.

Following is the amino acid sequence encoded by the preceeding DNA sequence: 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGlnValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg

AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
 wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, lle is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

Those skilled in the art will recognize that the DNA sequence depicted above is an important part of the present invention The above sequence can be conventionally synthesized by the modified phosphotriester method using fully protected deoxyribonucleotide building blocks. Such synthetic methods are well known in the art and can be carried out in substantial accordance with the procedure of Itakura et al., 1977, Science 198: 1056 and Crea et al., 1978, Proc. Nat. Acad. Sci. USA 75: 5765. In addition, an especially preferred method is disclosed in Hsiung et al., 1983, Nucleic Acid Research 11: 3227 and Narang et al._{;} 1980, Methods in Enzymology 68: 90. In addition to the manual procedures referenced above, the DNA sequence can be synthesized using automated DNA synthesizers, such as the ABS (Applied Biosystems, 850 Lincoln Centre Drive, Foster City, CA 94404) 380A DNA Synthesizer. The DNA sequence can also be generated by the polymerase chain reaction. See, e.g., U.S. Patents Nos. 4,800,159 and 4,683,202 and European Patent Publication No. 0258017, published March 2, 1987.

The amino acid sequence of the acyltransferase enzyme depicted above can be encoded by a multitude of different DNA sequences because most of the amino acid residues are encoded by more than one DNA triplet. Because these alternate DNA sequences would encode the same amino acid residue sequence of the present invention, the present invention further comprises these alternate sequences.

The present invention comprises DNA compounds and recombinant DNA cloning and expression vectors that encode the epimerase activity of Streptomyces lipmanii. The compounds can be adapted for use in any organism in which it is desired to produce the enzyme. One need only to insert an epimerase-encoding DNA sequence in an appropriate vector such that the coding sequence is under the control of a promoter and translational activating sequence functional in the organism An epimerase-activity-encoding DNA compound of the present invention was isolated from a strain of Streptomyces lipmanii. A genomic library of the total genomic DNA of the S. lipmanii strain was constructed and examined for the presence of sequences homologous to a deoxyribooligonucleotide probe. This probe was constructed in accordance with information obtained about the amino-terminal amino acid sequence of the S. clavuliqerus epimerase, with knowledge of the genetic code and codon usage preferences of Streptomyces, and potential cross-hybridization between genes encoding the epimerase enzymes of related organisms. DNA sequencing revealed which vector encoded the S. lipmanii epimerase.

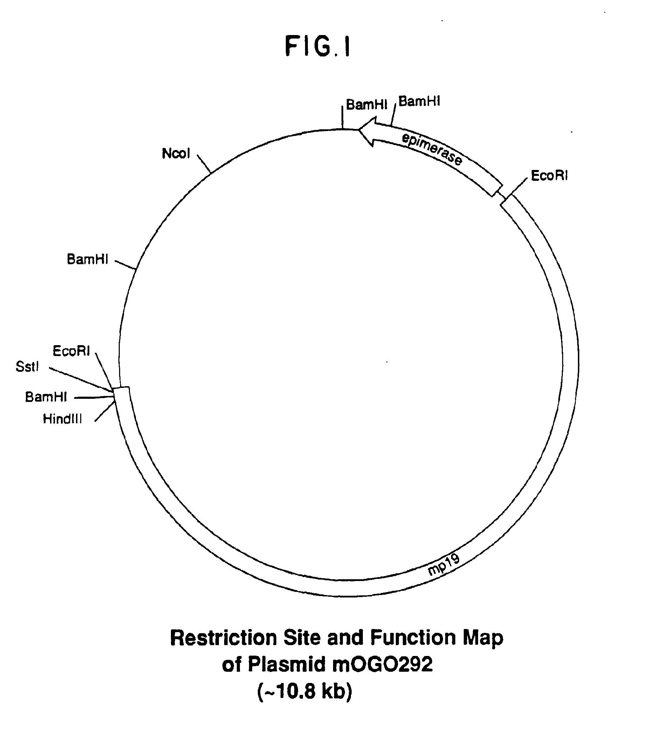

An ~3.5 kb EcoRl restriction fragment of a lambda EMBL3 S. lipmanii genomic library clone (designated as IOG02) was identified as comprising the epimerase gene. The fragment was inserted into commercially available double-stranded mp19 (digested with EcoRl; Bethesda Research Laboratories, Inc. P.O. Box 577 Gaithersburg, MD 20760) to yield plasmid mOG0292, which was then transformed into E. coli K12 JM109 host cells. The E. coli K12 JM109/mOG0292 transformants have been deposited and made part of the stock culture collection of the Northern Regional Research Laboratories (NRRL), Peoria, IL 61604, under the accession number NRRL B-18511 (date of deposit: June 19, 1989). A restriction site and function map of plasmid mOG0292 is presented in Figure 1 of the accompanying drawings.

Plasmid mOG0292 serves as useful starting material for the construction of other expression vectors of the invention. These vectors are especially useful in a method for constructing a recombinant host cell capable of expressing epimerase activity, said method comprising: transforming said host cell with a recombinant DNA expression vector that comprises: (a) a promoter and translational activating sequence; and (b) a DNA sequence encoding epimerase activity positioned for expression from said promoter and translational activating sequence. The vectors are also useful in a method for expressing epimerase activity in a recombinant host cell, said method comprising: culturing a host cell transformed with a recombinant DNA vector capable of expressing epimerase activity under conditions that allow for expression of said epimerase activity. If recovery of the enzyme is desired, purification of the protein may be accomplished via techniques well-known to one skilled in the art.

Plasmid mOG0292 can be isolated from E. coli K12 JM109/mOG0292 by the procedure described in Example 1. Plasmid mOG0292 contains the intact Streptomyces lipmanii epimerase gene which can be isolated, for example, from the plasmid on an -3.5 kb EcoRl restriction fragment. Plasmid mOG0292 was used as starting material in the construction of a plasmid, designated pOG0299, that drives high-level expression of epimerase activity in E. coli. To facilitate manipulation of the S. iipmanii epimerase coding sequence, a Ncol restriction enzyme recognition sequence was created at position -2 to +4 of the S. lipmanii epimerase DNA coding sequence in plasmid pOG0299.

The creation of the Ncol site was done by M13 site-directed mutagenesis techniques. DNA bases surrounding the start codon of the epimerase gene were changed from AAATGG to CCATGG. One skilled in the art will recognize that DNA mutagenesis techniques are commonly used to introduce restriction enzyme recognition sites into DNA sequences. In this case, the encoded amino acid sequence was not changed. The mutagenesis and intermediate constructs are described in qreater detail in Example 2.

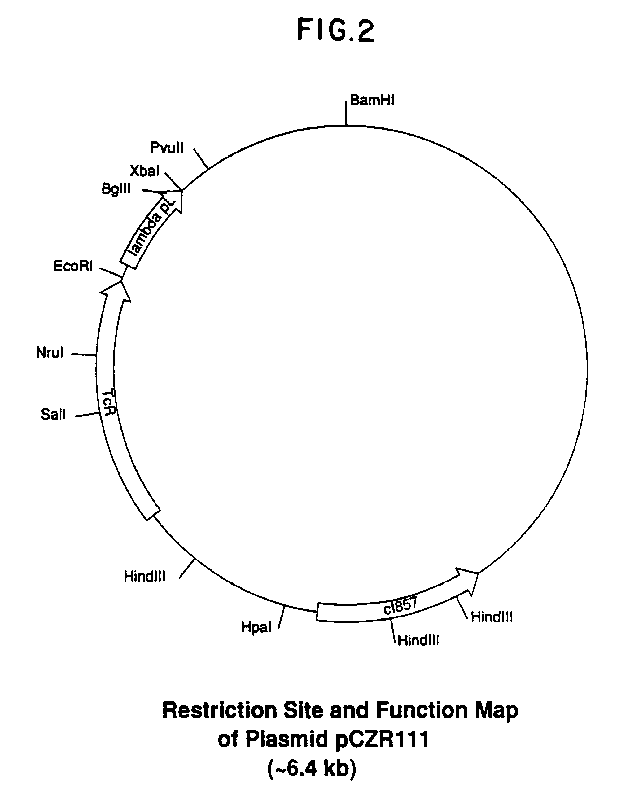

Plasmid pOG0299 can be constructed by inserting the Streptomyces lipmanii epimerase coding sequence into plasmid pCZR111 (available from the NRRL under accession number NRRL B-18249(date of deposit: August 11, 1987)), an expression vector that comprises the lambda pL (λpL) promoter and a translation activating sequence, the c1857 temperature sensitive repressor gene, a tetracycline resistance-conferring gene, and DNA sequences encoding vector replication functions. For a detailed description of the construction of pOG0299, see Example 2. TheaλpL-derived promoter of plasmid pCZR111 is positioned to drive expression of the epimerase gene. At low temperatures of about 30°C, the c1857 protein encoded on plasmid pCZR111 or its derivatives is active and able to repress activity of the ÀpL promoter, but when the temperature is raised to about 42°C, the c1857 protein is inactivated, and the λpL promoter drives transcription of large amounts of mRNA encoding the gene product of interest. A restriction site and function map of plasmid pCZR111 is presented in Figure 2 of the accompanying drawings.

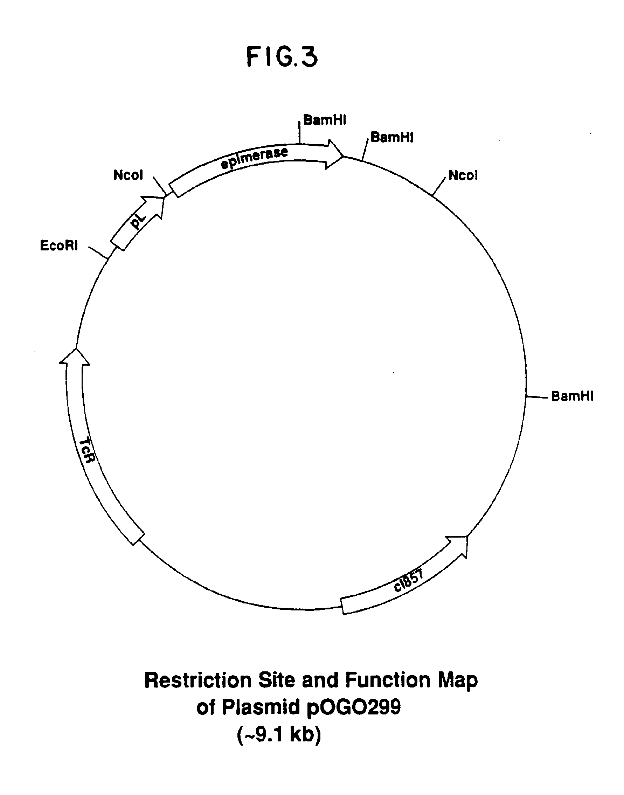

Plasmid pOG0299 comprises the same cl857 gene, λpL promoter, and translational activating sequence as does plasmid pCZR111 but further contains the coding sequence of the epimerase gene from plasmid mOG0292 positioned for expression from the ÀpL promoter. The ~3.5 kb EcoRl restriction fragment of plasmid mOG0292 comprises the complete coding sequence for epimerase. Plasmid pOG0299 was constructed so that the λpL promoter and the translational activating sequence of plasmid pCZR111 are positioned to drive expression of the epimerase-activity-encoding DNA. A restriction site and function map of plasmid pOG0299 is presented in Figure 3 of the accompanying drawings.

At temperatures of about 42°C, E. coli K12 JM109/pOG0299 expresses epimerase activity at high levels, approaching ~5% of the total cell protein. Crude cell extracts from these E. coli K12 JM109/pOGO299 transformants are able to catalyze the conversion of isopenicillin N into penicillin N, whereas cell extracts from non-transformed E. coli K12 JM109 cells cannot catalyze this conversion. The method of assay and results of the assay for the conversion reaction are presented in Example 4.

Plasmid pOG0299 provides an efficient means of producing large amounts of epimerase activity inE.coli. Because culturing E. coli is less complex than culturing organisms that naturally produce epimerase, E. coli/pOGO299 transformants can be used to produce recombinant epimerase more efficiently and economically than non-recombinant or "natural" epimerase producers.

The epimerase enzyme can be used to produce penicillin N from isopenicillin N in a cell-free system as described in Example 3. Penicillin N is not only a useful antibiotic, but can also be used as the starting material for the production of such important antibiotics as cephalexin and other cephalosporins (see U.S. Patent No. 4,307,192). Perhaps the most important use of epimerase is the use of the enzyme to transform penicillins other than isopenicillin N into novel antibiotic derivatives. An example would be to use cephamycin C as a substrate for epimerase, catalyzing the reverse reaction from its mode in natural biosynthetic pathways, to generate an L-aminoadipoyl derivative which could then serve as a substrate to form 7-aminocephalosporanic acid, a starting material for semi-synthetic cephalosporins.

Cell-free extracts of penicillin and cephalosporin-producing organisms can be used to synthesize unnatural (not produced in nature) β-lactams. The E. coli expression vectors of the present invention provide an inexpensive and efficient method of obtaining epimerase activity which can be used in vitro to modify penicillins to form novel antibiotics or antibiotic core structures.

Plasmid pOG0299 is especially preferred for driving expression of epimerase activity in E. coli not only because of the high expression levels achieved when using the plasmid but also because of the selectable marker present on the plasmid. Many recombinant DNA vectors encode a β-lactamase, so that cells containing the vector can grow in the presence of certain β-lactam antibiotics, such as ampicillin. However, if one desires to use a cell-free extract containing epimerase activity for purposes of constructing β-lactams, one does not want the extract to contain β-lactamase activity. Thus, plasmid pOG0299 does not encode a β-lactamase for a selectable marker but rather employs a tetracycline resistance-conferring gene, which encodes a protein that does not react with β-lactams.

The epimerase expression vectors of the present invention, however, are not limited to a particular selectable marker. Those skilled in the art recognize that many selectable markers are suitable for use on epimerase expression vectors Such selectable markers include, for example, genes that confer kanamycin resistance, genes that confer chloramphenicol resistance, or other antibiotic resistance-conferring genes.

As those skilled in the art will recognize, the present invention allows one to change the codons for the epimerase gene at will Given the DNA sequence for the epimerase gene, procedures familiar to one skilled in the art are used to generate mutant epimerase enzymes that vary from the natural epimerase enzyme at any number of amino-acid residue positions. Such mutant enzymes would be encoded by mutant epimerase coding sequences, including sequences in which amino-acid codons have been deleted from or inserted into the natural epimerase coding sequence. Such mutant enzymes are within the scope of the present invention, because even if one cannot absolutely predict whether a given mutation will destroy activity of the encoded epimerase, one need merely express the mutant sequence via the procedures provided herein to ascertain the effect on epimerase activity.

The present invention is not limited to the particular vectors exemplified herein. Instead, the present invention comprises DNA compounds that encode the epimerase activity of Streptomyces lipmanii. The DNA compounds of the present invention can be used to construct expression vectors that drive expression of epimerase activity in any host cell in which the expression vector replicates or integrates and in which the promoter and translational activating sequence are functional.

Therefore, the present invention comprises any E. coli plasmid or other vector that drives expression of epimerase activity in E. coli. The present invention comprises vectors that drive expression of epimerase activity and utilize a replicon functional in E. coli, such as, for example, a replicon from such plasmids as pBR322, pACYC184, F, CoIV-K94, R1, R6-5, or R100. Nor is the present invention solely limited to plasmid vectors, for the present invention also comprises other vectors that express epimerase activity and utilize integration or viral replication to provide for replication and maintenance in the host cell.

The present invention is not limited to a particular promoter and translational activating sequence to drive expression of the epimerase activity-encoding DNA. The present invention comprises the use of any promoter and translational activating sequence that function in E. coli and are used to express epimerase activity in E. coli. Many promoter and translational activating sequences functional in E. coli are known and are suitable for driving expression of epimerase activity in E. coli. Such transcriptional and translational activating sequences include, but are not limited to, the Ipp, lac, trp, tac, λpL, and ÀpR promoter and translational activating sequences.

In addition, transcriptional and translational activating sequences from other organisms can be ligated to the present epimerase-activity-encoding DNA compounds to form expression vectors that drive expression of isopenicillin N epimerase activity in host cells in which the activating sequence functions. Although E. coli is the host best suited for epimerase production and subsequent purification for in vitro use, vectors that drive expression of isopenicillin N epimerase activity in host cells other than E. coli are also useful, especially for purposes of increasing the antibiotic-producing ability and efficiency of a given organism.

A variety of organisms produce β-lactam antibiotics. The following Table presents a non-comprehensive list of P-lactam antibiotic-producing organisms.

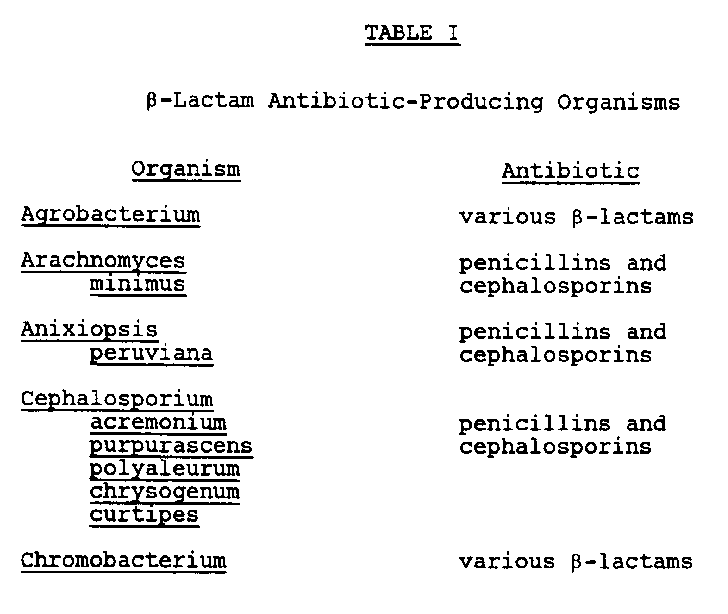

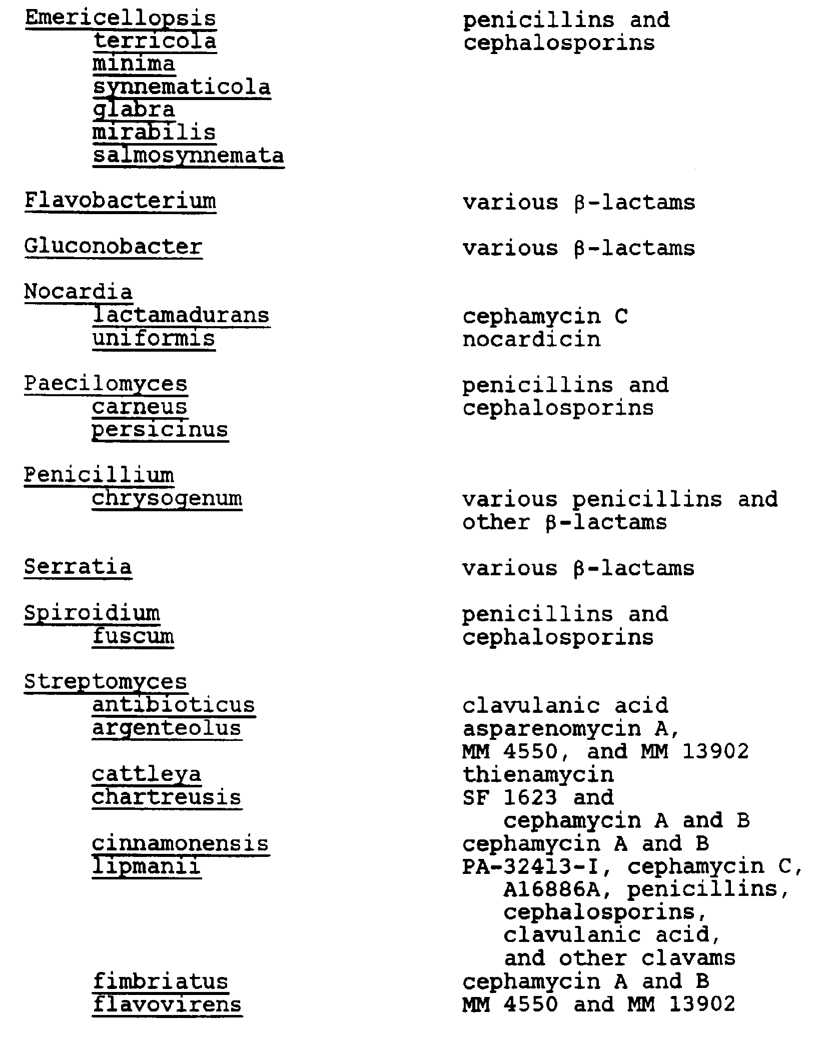

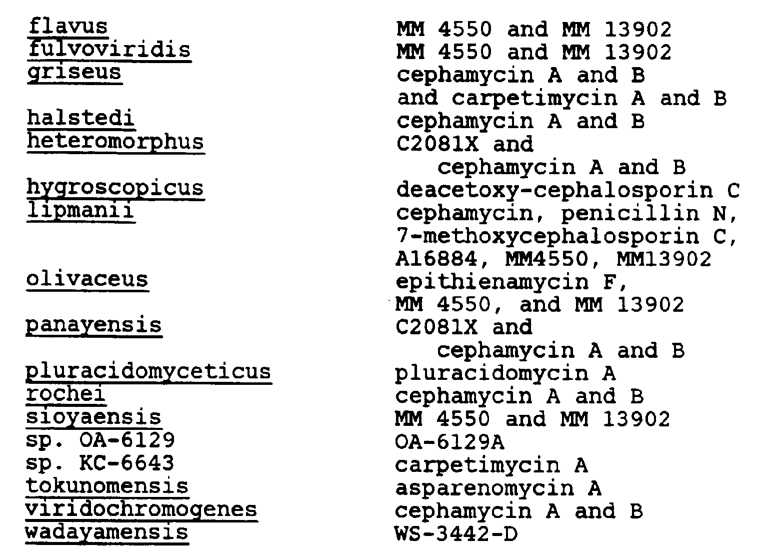

The antibiotic-producing ability of these organisms can be increased and made more efficient by increasing the intracellular concentration of the antibiotic biosynthetic enzymes during the fermentation. The epimerase activity-encoding DNA compounds of the present invention can be used to construct expression vectors that, when transformed into the appropriate host cell, increase the intracellular concentration of epimerase activity of the transformed host cell and thereby increase the antibiotic-producing ability and efficiency of that cell, provided that the host cell produces a β-lactam antibiotic via an intermediate reaction involving epimerase activity A preferred use of the compounds is to increase the yield of an antibiotic of industrial importance.

A vector that will increase the intracellular concentration of epimerase activity of a given host cell into which the vector is transformed requires the following elements: 1) an epimerase activity-encoding DNA compound of the present invention, and 2) a promoter and translational activating sequence that not only function in the host cell to be transformed, but also are positioned in the correct orientation and position to drive expression of the epimerase activity-encoding DNA. Of course, stable transformants can only be obtained if the vector replicates, either as an extrachro- mosomal element or integrated in the genomic DNA, in the host cell. Thus, a preferred vector contains sequences that specifically direct replication or integration of the vector in the host cell. However, the presence of such specific replication or integration sequences is not absolutely required, as non-specific integration may occur when DNA is introduced into a host cell. An epimerase expression vector could also comprise an antibiotic resistance-conferring gene or some other element that provides a means of selecting for host cells which contain the vector, but such selectable elements may neither be necessary nor desired when the vector integrates into the chromosomal DNA of the host cell.

By providing the coding sequence of the epimerase gene of Streptomyces lipmanii, the present invention provides epimerase expression vectors for any organism susceptible to transformation. The E. coli epimerase expression vectors described above illustrate the wide variety of expression vectors of the present invention. However, many of the preferred vectors of the invention are designed to drive expression of epimerase in a β-lactam antibiotic producing cell.

The Penicillium vectors of the invention are illustrative of the vectors provided by the present invention that can be used to increase the yield of antibiotic from such a β-lactam antibiotic-producing cell or to alter the antibiotic normally produced by the cell. Epimerase expression vectors that contain the acetamidase gene or the phleomycin resistance gene are particularly useful as vectors for inserting genes into Penicillium chrysoqenum because no special recipient strain, such as an auxotroph, need be constructed, owing to the natural inability of P. chrysoqenum to grow on acetamide as sole nitrogen source or in the presence of phleomycin. Transformation systems based on complementation of auxotrophic markers by a gene in the transforming plasmid do not share this advantage. Frequently, pleiotropic mutations are associated with the introduction of an auxotrophic marker into aP. chrysoqenum strain highly developed for penicillin production. Such mutations usually result in lower penicillin production (MacDonald et al., 1963, J. Gen. Microbiol. 33: 365-374).

European Patent Publication No. 0281391, published September 7,1988, describes the 5' and 3' regulatory signals of the Cephalosporium acremonium expandase-hydroxylase gene. The signals can be combined with the epimerase coding sequence of the present invention to yield epimerase expression vectors of the invention especially suited for use in Cephalosporium. US. Patent No. 4,885,251 discloses the transcription and translation activating sequences of the Cephalosporium acremonium IPNS gene, which can be fused to the Streptomyces lipmanii epimerase coding sequence of the present invention to create a recombinant epimerase gene that drives expression (when incorporated into an expression vector and the vector introduced into Cephalosporium) of the S. lipmanii epimerase coding sequence in Cephalosporium. The vectors described above and in the Examples are merely illustrative of the wide variety of epimerase expression vectors provided by the present invention.

The epimerase expression vectors of the present invention are useful for increasing the intracellular concentration of epimerase activity in any cell, especially β-lactam antibiotic-producing cells. Plasmid mOG0292 comprises the coding sequence of the epimerase gene of Streptomyces lipmanii, so plasmid mOG0292 can be used to construct vectors for increasing the copy number of the epimerase gene and thus for increasing intracellular concentration of the enzyme. Because the epimerase coding sequence of the invention was isolated from a Streptomyces host cell, the epimerase coding sequence is particularly well-suited for use in expression vectors designed to drive high-level expression of epimerase activity in Streptomyces host cells. The literature is replete with techniques for constructing Streptomyces expression vectors and for transforming Streptomyces host cells. See, for instance, Garcia-Dominguez et al., 1987, Applied and Environmental Microbiology 53(6): 1376-1381. The epimerase coding sequence of the invention can be readily incorporated into an expression vector that comprises a Streptomyces promoter and replicon. A variety of known Streptomyces promoters and replicons are available for such use. Table II is an illustrative, but not comprehensive, listing of Streptomyces plasmids from which Streptomyces replicons can be obtained. Those skilled in the art recognize that, so long as the replicon function is not disrupted, all or part of the plasmids listed in the Table may be used to construct vectors that contain the epimerase gene of the present invention. The plasmid-containing host and depository accession number are also listed in Table II.

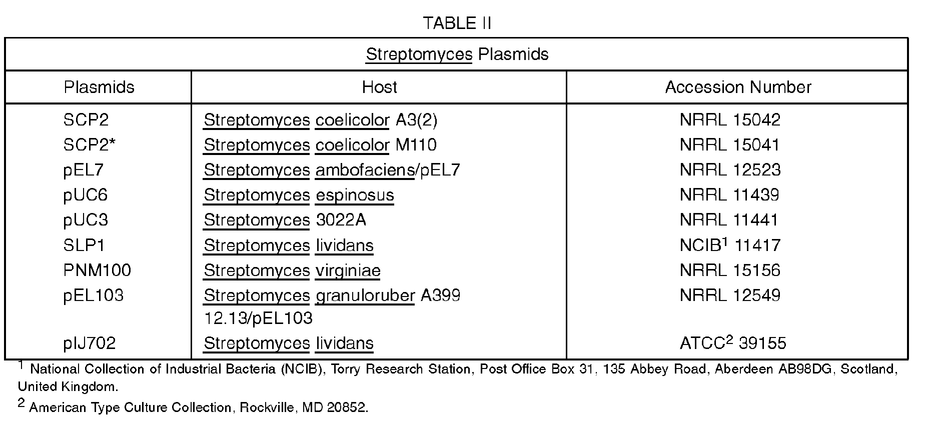

The Streptomyces lipmanii epimerase coding sequence of the invention can also be put under the control of transcription and translation activating sequences derived from other strains of Streptomyces, as well as from Penicillium, Cephalosporium, or any other host cell to construct a recombinant epimerase gene for use in the given organism.

The following Examples are provided to further illustrate and exemplify, but not to limit the scope of, the present invention.

### Example 1

### Source of the S liomanii epimerase gene

### Preparation of a Phage Stock Solution of mOG0292

A lyophil of E. coli K12 JM109/mOG0292 can be obtained from the Northern Regional Research Laboratories (NRRL), Peoria, IL 61604, under the accession number NRRL B-18511 (date of deposit: June 19, 1989) and used directly as the "culture" in the process below. A restriction site and function map of plasmid mOG0292 is presented in Figure 1 of the accompanying drawings. Ten ml of TY broth (10 g tryptone, 5 g NaCI, and 5 g yeast extract per liter) is inoculated with a culture of E. coli K12 JM109/mOG0292 and incubated with aeration at 37°C overnight (15-18 hours). The resulting culture is used as a source of plasmid mOG0292.

A series of 1: 10 dilutions of the culture is made into 2X TY broth. One hundred µl of each dilution is placed into a separate capped 5 ml tube. Added to each tube is 200 µl E. coli JM1 09 grown to saturation and 3 ml top agar (TY broth + 0.6% agar kept molten at 45°C). The tubes are rapidly inverted twice and the contents are poured onto TY agar plates (TY broth + 1.5% agar) prewarmed to 37°C. The top agar is allowed to harden for 10 minutes at room temperature. The plates are then incubated overnight at 37°C.

Plaques are plugged from the plate overlay with a Pasteur pipette into 3 ml per plaque of TY broth. Cultures are incubated from 6 to 18 hours at 37°C with aeration. Following this incubation, 1.5 ml of each culture are pelleted in separate 1.5 ml Eppendorf tubes. The supernatants are decanted into fresh tubes and stored at 4°C to serve as a source of phage inoculum.

### Example 2

### Construction of Plasmid mOG0297

The M13-derived plasmid mOG0292 was used to construct intermediate plasmid mOG0297. Site-specific mutagenesis was employed to create an Ncol restriction enzyme recognition site at the 5' end of the S. lipmanii epimerase coding sequence, resulting in plasmid mOG0297. The coding sequence and 3' untranslated region was then isolated and ligated to the vector backbone of plasmid pCZR111, thus creating E. coli expression plasmid pOG0299.

### A. Preparation of Single-Stranded Plasmid mOG0292 DNA and Site-Specific Mutagenesis to Construct Plasmid mOG0297

A 10 ml culture of early logarithmic growth phase E. coli K12 JM109 was inoculated with ~200 µl of mOG0292 phage stock (as prepared in Example 1) and incubated ~18 hours at 37°C with aeration. The culture was pelleted by centrifugation at 5200 rpm in a Sorvall GSA rotor (DuPont Co., Instrument Products, Biomedical Division, Newtown, CT 06470) for 10 minutes at 4°C and the resulting supernatant was transferred to a new tube and recentrifuged. The supernatant was again decanted to a fresh tube. One ml of a solution of 25% polyethylene glycol (molecular weights 3,350) in 3 M NaCl was added to the supernatant, which was then incubated for 15 minutes at room temperature The resulting mixture was centrifuged for 30 minutes at 10,000 rpm in a Sorvall SS34 rotor. The pellet obtained by the centrifugation contained the single-stranded plasmid mOG0292 and was resuspended in 400 µl of TE buffer. TE buffer is 10 mM Tris-HCI (Tris(hydroxymethyl)amino-methane hydrochloride), pH = 7.5, and 1 mM EDTA (ethylenediamine tetraacetic acid). The solution was extracted first with CHC1₃ and then with TE-saturated phenol. The phenol was allowed to stay in contact with the aqueous phase for 15 minutes. The solution was then extracted twice with a mixture of TE-saturated phenol: CHCl₃ (1: 1, v/v), and twice with CHCI₃ alone. The DNA was then precipitated out of 0.3 M sodium acetate in 70% ethanol, collected by centrifugation, and the resulting pellet resuspended in 100 µl of 0.1 X TE buffer. This solution constituted ~5 µg of single-stranded plasmid mOG0292 DNA.

### B. Mutagenesis

The single-stranded DNA fragments used in the mutagenesis (and subsequent hybridizations to detect desired phages) were synthesized on an automated DNA synthesizer such as the ABS 380A synthesizer (Applied Biosystems, 850 Lincoln Centre Drive, Foster City, CA 94404), with the exception of the M13 universal primer (a 15-mer), which was purchased from BRL Bethesda Research Laboratories, Inc., P.O. Box 577, Gaithersburg, MD 20760). The mutagenesis fragments were designated as follows: (1) RACE-10, a single-stranded DNA 39 nucleotides in length that is homologous to the epimerase coding sequence in plasmid mOG0292 except for 2 bases, the mismatch (underlined) of which will create a restriction enzyme Ncol recognition sequence at about position 1 of the epimerase coding sequence, with the DNA sequence: 5'-GAGTGTTCGGGAGATCACGCCATGGCCTGTTCCGACTGG-3' (2) RACE-11, a single-stranded DNA 17 nucleotides in length that is a subfragment of RACE-10 with the DNAsequence:

NcoI
5'-GATCACGCCATGGCCTG-3'

The 5' ends of about 100 pmols of RACE-10 were phosphorylated (kinased) in a reaction mixture containing 4 µl single-stranded DNA at a final concentration of 1 pmol/µl, 10 µl of 10X ligase buffer (0.5M Tris-HCl, pH 7.5 and 100 mM MgCl₂), 10 µl 0.1 mM adenosine triphosphate (ATP), 10 µl of 0.1 M DTT, 65 µl of glass-distilled water, and 1 µl (10 Richardson units)of T4polynucleotide kinase (Boehringer-Mannheim Bio-chemicals, (BMB) 7941 Castleway Drive, P.O. Box 50816, Indianapolis, Indiana 46250). The reaction mixture was incubated at 37°C for 30 minutes, at which time an additional 1 µl of enzyme was added. The reaction mixture was then incubated for another 30 minutes at 37°C. The 5' ends of about 40 pmols of M13 universal primer were kinased in an analogous 40µl of reaction mixture containing the same amount of enzyme.

The single-stranded plasmid mOG0292 DNA was mutagenized in substantial accordance with the teaching of U. S. Patent No 4,885,251, herein incorporated by reference, as described below The annealing reaction was carried out by adding ~500 nanograms (in 15 µl of 0.1 X TE buffer) of single-stranded plasmid mOG0292 DNA to 4 µl of buffer (100 mM Tris-HCI, pH =7.5; 1 mM EDTA; and 500 mM NaCI), 6 µl (6 pmols) of kinased RACE-10, 4 µl (4 pmols) of kinased M13 universal sequencing primer, and 18 µl of water, incubating the mixture at 90°C for 2 minutes, then at 55°C for 5 minutes, and finally at room temperature for 5 minutes.

The extension reaction was carried out by adding 60 µl of the following mixture to the solution of annealed DNA: 6 µl Klenow-ligase buffer (100 mM Tris-HCI, pH = 7.5; 1 mM EDTA; and 500 mM NaCI), 6 µl of 0.1 M DTT; 6 µl of a solution 6.25 mM in each of dGTP, dATP, TTP, and dCTP; 6 µl of 5 mM ATP; 36 µl of water; 3 µl (3 Weiss units, BMB) ligase; and 2 pl (10 units) of Klenow enzyme (BMB). The extension reaction mixture was incubated at room temperature for 1 hour, then at 37°C for 4 hours, and finally at 14°C for~18 hours.

The extension reaction mixture was extracted once with CHCl₃ and the DNA precipitated with ethanol and sodium acetate and collected by centrifugation. The DNA pellet was resuspended in 40 µl S1 buffer (0.3 M NaCl and 3 mM zinc acetate and 1 µl (400 units) of S1 nuclease (BMB). The reaction was incubated at room temperature for 15 minutes. The reaction was stopped by first adding 5-10 µg of tRNA to the reaction mixture to serve as carrier, then extracting with TE-saturated phenol The aqueous phase was then extracted again with CHCl₃. The DNA in the aqueous phase was concentrated by ethanol precipitation and collected by centrifugation. The DNA pellet was resuspended in 20 µl water.

Competent E. coli K12 JM109 ("Epicurean Coli™") were purchased from Stratagene (3770 Tansy Street, San Diego, CA 92121) and transformed with 10 µl of the resulting S1-treated DNA solution. The ligated DNA was mixed with 100 µl of the cells, incubated for one hour on ice, then incubated for one minute at 42°C. The cells were then distributed in ~1, 10, 20, 40 and 50 ul samples to 13 X 100 mm sterile glass tubes containing 0.25 ml per tube ofE. coli K12 JM109 in logarithmic growth phase. To these tubes were added 3 ml of top agar. The cell-top agar mixture was then plated on TY-agar plates, and the plates were incubated at 37°C overnight. (For more detailed descriptions and explanations of M13 procedures, see M13 Cloning/Dideoxy Sequencing Instruction Manual, Bethesda Research Laboratories (BRL), Life Technologies, Inc., Gaithersburg, MD 20877.)

Desired mutants were identified by hybridization of radiolabelled oligonucleotide RACE-11 with plasmid DNA blotted onto nitrocellulose filters as described below. After plaque formation, the plates were incubated at 4°C for~ 1 hour to harden the top agar. Nitrocellulose filters were placed on top of the lawn of each of four plates, containing ~50-200 plaques, of the 15 minute S1-treated series Contact between the filter and the surface of the lawn was maintained for -1 minute, at which time the nitrocellulose filter was treated, by using 3MMChr filter papers (Whatman LabSales, Inc., P.O. Box 1359, Hillsboro, Oregon 97123-1359) saturated with 0.1 N NaOH-1.5 M NaCl for -5 minutes, then 0.5 M Tris-HCl(pH 7.0)-3 M NaCl for ~5 minutes. The nitrocellulose filters were air-dried and then baked in a vacuum oven at 80°C for 30 minutes.

Prehybridization was performed by treating the nitrocellulose filters for ~5 minutes at room temperature in a solution of 6X SSC (20X SSC is 3 M NaCl and 0.3 M sodium citrate), 10X Denhardt's solution (0.2 g of polyvinylpyrolidone, 0.2 g of bovine serum albumin, and 0.2 g of Ficoll per 100 ml of water), 0.1% sodium pyrophosphate, 0.1% SDS (sodium dodecyl sulfate), and 10 µg/ml of denatured E. coli chromosomal DNA. The filters were then treated for 90 minutes at 56°C in a solution of 6X SSC, 10X Denhardt's solution, 0.1 % sodium pyrophosphate, and 1 pmol/5 ml of ^{32p-}RACE-11. The ^{32p-}RACE-11 was prepared by phosphorylating the 5' ends of 100 pmols of RACE-11 in substantial accordance with the procedure described earlier in this example, exceptthat ~120 pmol of γ-³²P-ATP (New England Nuclear (NEN), 549 Albany Street, Boston, MA, 02118, Catalog # NEG-002A) were used instead of non-radioactive ATP. After hybridization, the filters were rinsed twice for 5 minutes per wash in excess 6X SSC at room temperature, then at 56°C in excess 6X SSC for 30 minutes. The filters were air-dried and autoradiographed for~18 hours at -70°C with a Quanta III® intensifying screen (DuPont, Instrument Products, Biomedical Division, Newtown, CT 06470). Desired mutants (those containing sequences complementary to the sequence of RACE-11) exposed the film due to binding of the radiolabelled oligomer by the M13 phage DNA bound to the filter. The identity of a correct mutant, designated plasmid mOG0297, was confirmed by restriction analysis of its replicative form (RF) DNA, which was prepared as follows.

Plaques are plugged from the plate overlay with a Pasteur pipette into 3 ml per plaque of TY broth. Cultures are incubated from 6 to 18 hours at 37°C with aeration. Following this incubation, 1.5 ml of each culture are pelleted in separate 1.5 ml Eppendorf tubes. The supernatants are decanted into fresh tubes and stored at 4°C to serve as a source of phage inoculum. Replicative form DNA is prepared from the cell pellets in substantial accordance with the teaching of the alkaline plasmid preparation procedure of Birnboim and Doly, 1979, Nuc. Acid Res. 7(6): 1513-1523, with the following exceptions. The procedure is scaled up such that 1.5X volumes of all solutions are used, and the cleared lysate is extracted once with an equal volume of CHCI₃. The DNA is then precipitated by the addition of 0.4 volumes of isopropanol and incubation at room temperature for 20 minutes. The DNA is collected by centrifugation and then precipitated with 70% ethanol out of 0.3 M sodium acetate. The DNA isolated from individual plaques was analyzed for the presence of a desired insert by Ncol-BamHl restriction enzyme digestion.

### Example 3

### Construction of Plasmid pOG0299

### A. Isolation of the ~3.3 kb Ncol-Bam Restriction Fragment of mOG0297

The RF DNA of plasmid mOG0297 contains the epimerase coding sequence on the Ncol-BamHl partial restriction fragment utilized in the construction of the E. coli expression plasmid pOG0299. Replicative form DNA from plasmid mOG0297-infected E. coli K12 JM109 was isolated in substantial accordance with the procedure described below

One liter of TY broth is inoculated with a culture of E. coli K12 JM109/mOG0297 and incubated with aeration at 37°C overnight (15-18 hours). The culture is then centrifuged at 5200 rpm in a Sorvall GSA rotor for 10 minutes at 4°C to pellet the cells. The resulting supernatant is discarded. The cell pellet is resuspended in 28 ml of a solution of 25% sucrose and 50 mM EDTA. About 1 ml of a solution of 20 mg/ml lysozyme in 50% glycerol and 0.25 M Tris-HCI, pH = 8.0, and about 1.5 ml of 0.5 M EDTA, pH 8.0, are added to and mixed with the cell suspension. The resulting mixture is incubated on ice for 15 minutes. Three ml of lysing solution (prepared by mixing 3 ml of 10% Triton X-100; 75 ml of 0.25 M EDTA, pH = 8.0; and 7 ml of water) are added to the lysozyme-treated cells with gentle mixing. The resulting solution is incubated on ice for another 15 minutes.

The cellular debris is removed from the solution by centrifugation at 17,000 rpm in a Sorvall SS34 rotor for about 45 minutes at 4°C. About 28.6 g of CsCl and ~1 ml of a 5 mg/ml ethidium bromide solution are added to the ~30 ml of supernatant. Then, the volume is adjusted to 40 ml with water and the solution decanted into an ultracentrifuge tube. The tube is sealed, and the solution is centrifuged at 49,500 rpm in a T170 rotor (Beckman, 7360 N. Lincoln Avenue, Lincolnwood, IL 60646) for~18 hours. The resulting plasmid band, visualized with ultraviolet light, is isolated, extracted with CsCI-saturated isopropanol to remove the ethidium bromide, and dialysed against three changes of ~20 volumes of TE buffer (10 mM Tris-HCI, pH = 7.5, and 1 mM EDTA). The dialysate is collected; then, two volumes of ethanol and 0.05 volumes of 3 M sodium acetate solution are added. The ethanol mixture is cooled to -20°C, and the plasmid DNA is pelleted by centrifugation at 10,000 rpm for 30 minutes in an SS34 rotor at -10°C. The resulting pellet is resuspended in ~1 ml of TE buffer and then extracted with an equal volume of a phenol: chloroform mixture (1: 1, v/v). The DNA in the aqueous phase is recovered by the addition of 0.1 volume of 3 M sodium acetate and 2 volumes of ethanol, followed by incubation at -20°C for~30 minutes and centrifugation at 15,000 rpm in an SS34 rotor for 20 minutes. The resulting DNA pellet is rinsed first with 70% ethanol and then with 100% ethanol and dried.

The~-1.0 mg of plasmid mOG0297 DNA obtained by this procedure is suspended in 1.5 ml of 0.1 X TE buffer and stored at -20°C. About 10 µg of the RF DNA of plasmid mOG0297 DNA are partially digested with restriction enzyme Ncol (-10 units) in a reaction containing the DNA in Ncol buffer (50 mM Tris-HCI, pH = 8.0, 10 mM MgCl₂, 100 mM NaCI). After incubation for ~2 minutes at 37°C, the restriction enzyme BamHl (-25 units) is added and incubation at 37°C continued for~2 minutes. The reaction mixture is extracted with CHCI₃, ethanol precipitated, subjected to agarose gel electrophoresis, and the ~3.3 kb Ncol-_BamHl partial digestion fragment is isolated using the EpiGene D-GeITM electroeluter (EpiGene, P.O. Box 4817, Baltimore MD 21211) in substantial accordance with manufacturer's directions.

### B. Isolation of the ~5.75 kb XBal Hl Restriction Fragment of pCZR111

Plasmid pCZRlll is available in E. coli K12 JM109 from the NRRL under accession number B-18249 (date of deposit: August 11, 1987). It can be isolated in substantial accordance with Example 3A except that 15 µg/ml tetracycline is included in the culture medium rather than 100 µg/ml ampicillin. A restriction site and function map of pCZR111 is presented in Figure 2.

The vector is generated by digesting plasmid pCZRlll with Xbal and BamHl. About 1 µl of Xbal (-10 units) is added to 10 µl plasmid pCZR111 (~10 µg) and 5 µl 10X Xbal buffer (500 mM Tris-HCI, pH=8.0, 100 mM MgCl₂, and 500 mM NaCI). After incubation at 37°C for 90 minutes, 0.5 µl of 5 M NaCl and 1 µl BamHl (-10 units) is added and the incubation continued at 37°C for an additional 90 minutes. The reaction mixture is then subjected to agarose gel electrophoresis, and the ~5.75 kb Xbal-BamHl vector fragment is isolated in substantial accordance with Example 3A.

### C. Final Construction of Plasmid pOG0299

Plasmid pOG0299 can be created by ligating the -3.8 kb BamHl-Ncol fragment from mOG0297, the -5.75 kb Xbal-BamHl vector fragment from pCZRlll and a double-stranded Xbal-Ncol restriction fragment synthesized by the phosphotriester method to yield the ~9.1 kb plasmid pOG0299. The double stranded DNA fragment constituting the 2 cistron translational activating sequence has the following sequence: 
5'-CTAGAGGGTATTAATAATGTATCGCGATTTAAAATAAGGAGGAATAATCC-3'
||||||||||||||||||||||||||||||||||||||||||||||
3'-TCCCATAATTATTACATAGCGCTAAATTTTATTCCTCCTTATTAGGGTAC-5'
 About 0.1 µg of the ~3.3 kb Ncol-BamHl restriction fragment of plasmid mOG0297, from Part D above, ~0.1 µg of the ~5.75 kb Xbal-BamHl restriction fragment of plasmid pCZR111, from Part E above, and ~1 pmol of the double-stranded Xbal-Ncol synthetic restriction fragment are ligated in a 20 µl reaction containing the DNA fragments, 2 µl of 10X ligase buffer (0.5M Tris-HCI, pH 7.5, and 100 mM MgCl₂), 2 µl of 5 mM ATP, 1 µl 6 µg/ml bovine serum albumin (BSA), 1 µl T4 DNA ligase (1 Weiss unit, BMB) and enough water to bring the reaction volume to 20 µl. The reaction is incubated for about 18 hours at 15°C. The ligated DNA constituted the desired plasmid pOG0299 along with other ligation products.

The ligation mixture is transformed into E. coli K12 JM109 as follows. Competent E. coli K12 JM109 ("Epicurean Coli™") are purchased from Stratagene and transformed with a ligation reaction mixture comprising plasmid pOG0299. The ligated DNA is mixed with 100 µl of competent JM109 cells, incubated for one hour on ice, then incubated for one minute at 42°C, then diluted with 2 ml of fresh TY broth and incubated at 37°C for one hour.

Aliquots of the transformation mixture were plated on TY-agar plates containing tetracycline (15 µg/ml). The plates were incubated at 37°C for~ 18 hours. Tetracycline-resistant transformants were further screened by restriction enzyme analysis of their plasmid DNA to identify the desired plasmid pOG0299 transformants. Plasmid DNA was prepared from 3 ml cultures in substantial accordance with the Birnboim and Doly procedure described above for preparing RF DNA from phage M13-infected E. coli K12 JM109 cell pellets. Plasmid pOG0299 DNA from one transformant was prepared in substantial accordance with the procedure described in Example 3A for use in subsequent constructions.

### Example 4

### Assay of E. coli-produced Epimerase Activity

### A. Culture of E.coli K12 JM109/pOG0299 for Expression of Epimerase Activity

An E. coli K12 JM109/pOG0299 transformant was grown at 30°C overnight in 400 ml of TY broth (containing 10 µg/ml of tetracycline) in a gyratory incubator. The cells were diluted 1: 10 by adding 100 ml of the overnight culture to 900 ml of fresh medium containing 10 µg/ml tetracycline in a 2.8 liter Fernbach flask. The temperature of the air shaker was then raised to 42°C and incubation continued for an additional 6 hours. The cl857 temperature-sensitive repressor of the lambda pL promoter, positioned to drive epimerase expression on plasmid pOG0299, is inactivated at 42°C, thus allowing the expression of epimerase. After induction, the cells were harvested by centrifugation and used as a preferred source of E. coli-produced epimerase activity.

### B. Demonstration of Isopenicillin N Epimerase Activity in the F coli K12 JM109/pOG0299 Cells Grown at 42°C

Five grams of the induced, pelleted E. coli were mixed with 10 ml of Breaking Buffer (10 mM sodium pyrophosphate-HCI, pH=8.0 in 5 M urea, 20% glycerol, and 0.1 mM dithiothreitol (DTT)) If the amount of cells after centrifugation was less than 5 gm, the ratio of cells to buffer was maintained. The cell suspension was then cooled to 2°C in an ice-ethanol bath and sonicated at full power for 20 seconds. The cooling and sonicating was repeated three times. Cell debris was removed by centrifugation at 47,000 x g for 20 minutes at 4°C. The supernatant was then filtered through glass wool with the filtrate being the crude enzyme.

The enzyme catalyzes the conversion of isopenicillin N to penicillin N and vice-versa, creating an equilibrium state between the two molecules. Therefore, the activity was measured in two ways, once using isopenicillin N as the initial substrate, and once using penicillin N as the starting material. Total protein was measured by the method of Lowry et al., J. Biol. Chem. 193, 265 (1951). Cell extract was mixed with 1.4 mM isopenicillin N or penicillin N, 0.2 mM dithiothreitol, 0.1 mM pyridoxal 5'-phosphate (as measured by the hydrazine method (Wada, H. and Snell, E., J. Biol. Chem. 236, 2089 (1961)), and 50 mM pyrophosphate-HCI, pH=8.3 in a final volume of 0.5 ml. The reaction mixture was then incubated at 37°C for 20 minutes. The reaction was terminated by boiling for 10 minutes. It was then put through a 0.45 µm filter.

Penicillin N and isopenicillin N are difficult to separate by HPLC. The compounds must be derivatized with o-phthaldialdehyde according to the method of Aswad, D.W., Anal. Biochem. 137, 405 (1984) and Usher, J., Lewis, M., and Hughes, D.W., Anal. Biochem. 149, 105 (1985). Twenty µl of the filtrate were mixed with 5 µl of the derivatizing solution (4 mg o-phthaldialdehyde (Sigma Chemical Co., P.O. Box 14508, St. Louis, MO 63178) dissolved in 300 pl methanol, 250 µl 0.4 M sodium borate, pH=9.₄, 390 µl H₂0 and 60 µl 1 M N-acetyl-L-cysteine, adjusted to pH 5-6 with NaOH). The reaction was allowed to proceed for 2 minutes at room temperature before termination with 200 µl 50 mM sodium acetate, pH=5.0. Fifty pl aliquots were analyzed by HPLC using a flow rate of 1 ml/min and measurement of fluorescence at 360 nm. When isopenicillin N was included in the mixtures the cell extracts expressing the epimerase activity converted isopenicillin N to penicillin N. Likewise, penicillin N was converted to isopenicillin N in the presence of these cell extracts. In each case a state approaching equilibrium was reached. No conversion was seen when the cell extract was not added to the mix. In addition, antibody to the purified epimerase enzyme reacted with an ~50,000 dalton band on a Western blot Taken together, these results indicate that the gene cloned results in a protein which has epimerase activity.

### Example 5

### Genetic Transformation of Penicillium

### A. Penicillium chrysogenum Strains

A Penicillium strain for transformation is obtained from the American Type Culture Collection, Rockville, MD 20852, under the accession number ATCC 9480. Other Penicillium chrysopenum strains or any commercial strains derived from ATCC 9480 by mutation, selection, or genetic breeding for the purpose of improved production of penicillin G or penicillin V are also suitable for use in preparing transformants with the vectors and plasmids of the present invention.

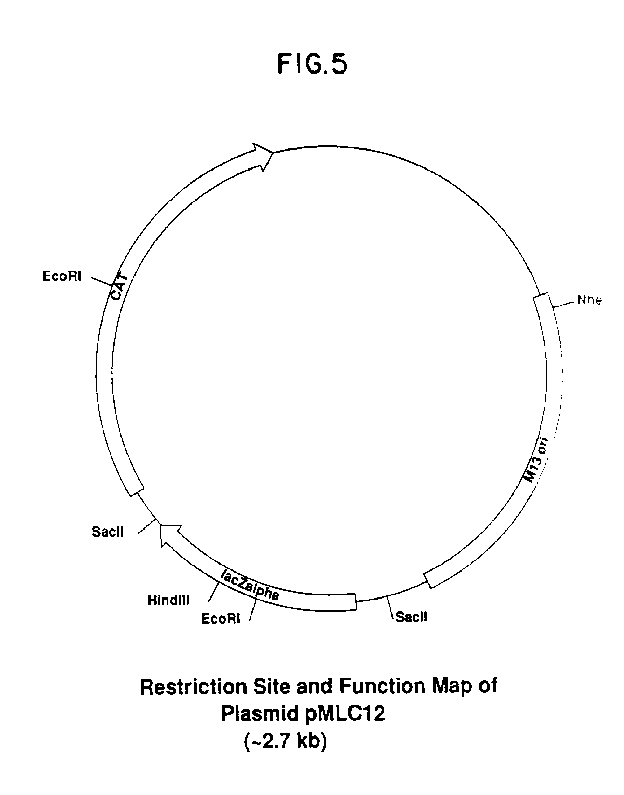

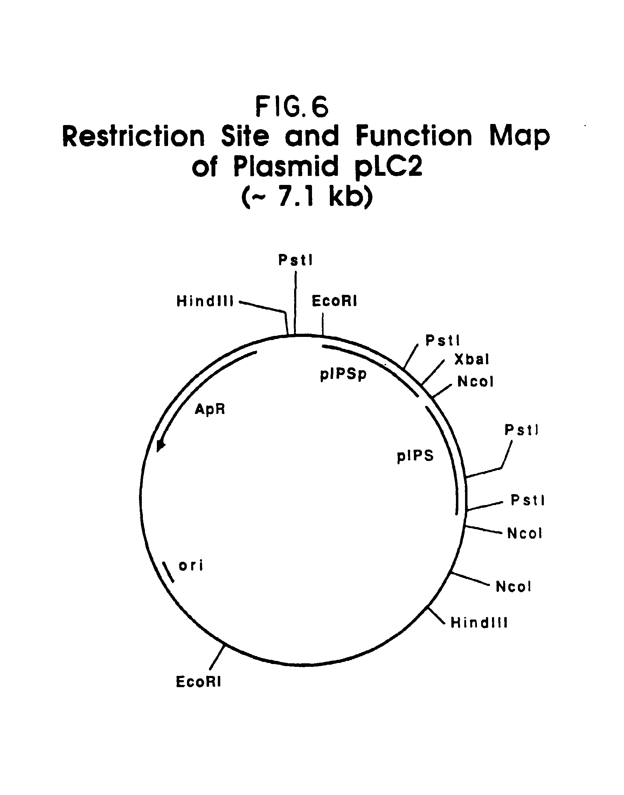

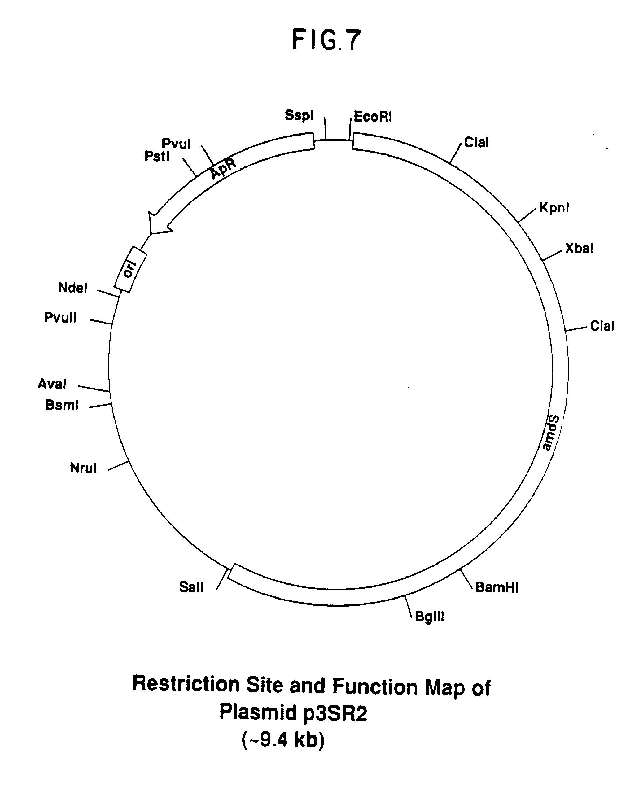

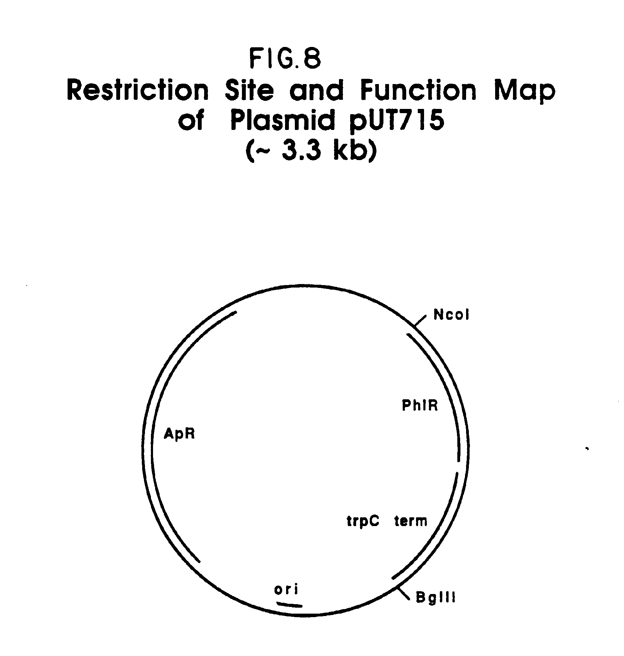

Any of the publicly available vectors suitable for use in Penicillium such as plasmid pMLC12 (NRRL B-18097 (date of deposit: August 8, 1986) and Figure 5) may be used to exploit the S. lipmanii epimerase gene in Penicillium. The gene may be put under the control of a promoter such as Penicillium chrysogenum IPNS promoter on plasmid pLC2 (ATCC 53334 and Figure 6). It is useful to use a vector comprising a selective marker such as the acetamidase gene (amdS) (available as an -5.0 kb _EcoRl-Sall restriction fragment of plasmid p3SR2 (NRRL B-18182 (date of deposit: February 26, 1987) and Figure 7). Alternatively the phleomycin resistance gene (phlR) may be obtained from Cayla, Avenue de Larrieu, 31094 Toulouse, Cedex, France on plasmid pUT715 (Figure 8). Phleomycin is also available from Cayla. The antibiotic should replace acetamide in this Example at a concentration of 1 mg/l.

### B. Preparation of Uniform Inoculum for Cell Culture

To transform Penicillium chrysogenum cells efficiently, it is necessary to remove the cell walls to form stable protoplasts. In the preparation of such protoplasts it is advantageous to begin with a uniform inoculum. Otherwise, preparation of cells in culture is not reproducible and time is lost by attempts to prepare P. chrysogenum protoplasts from unsuitable or inadequate amounts of cells.

An ampoule of vegetative cells (~10⁹ colony forming units in 1.0 ml of preservation menstruum: 5% lactose, 10% glycerol, and 0.1 % Tween 80), either lyophilized or taken from liquid nitrogen storage and thawed at room temperature, are diluted in 1.0 ml of sterile saline. About 0.1 ml of this suspension is used to inoculate each of approximately 20 slants of sporulation medium: lactose, 15.0 g/l; corn steep liquor, 2.5 g/l; peptone, 5.0 g/l; NaCI, 4.0 g/l; MgS0₄.7H₂0, 0.5 g/l; KH₂PO₄, 0.6 g/l; FeCl₃.6H₂0; 0.005 g/l; CuSO₄.5H₂O, 0.002 g/l; adjust to pH=₇.0, agar, 30.0 g/l; and autoclave 20 minutes at 120 psi.

Each slant [15 cm x 2.5 cm] contains 25 ml of solidified medium. Inoculum, spread evenly over the surface of the agar slant, is allowed to grow at 25°C until a confluent lawn of mycelium is present and sporulated (1 week for most strains). The growth from 1 slant is suspended in 10 ml of sterile aqueous culture medium, and the suspension is transferred to 106 ml of aqueous culture medium. The flask containing the suspended cells is placed on a gyratory shaker and incubated at 25°C for 18 hours at 285 rpm with a 1 inch throw.

Aqueous culture medium was prepared as follows: 100 ml of solution A (sucrose, 36 g/l; L-asparagine, 7.5 g/l; KH₂PO₄, 15 g/l; K₂HP0₄, 21 g/l; NaS0₄, 0.75 g/I, MgSO₄·7H₂O; 0.18 g/l; CaCl₂, 0.06 g/I; salts solution, 1 ml/l; and natural pH) are dispensed into a 500 ml shake flask. Salts solution is (per liter): 15 g Fe(NH₄)(SO₄)₂.6H₂O; 3 g MnSO₄·4H₂O; 3 g ZnSO₄·7H₂O; and 0.8 g CuSO₄·5H₂O. The flask is then covered with a commercial closure and autoclaved at 121°C for 20 minutes. Two ml of solution B (glucose, 108 g/I) and 4 ml of solution C (sucrose, 25 g/I; corn steep liquor (4% w/v nitrogen), 12.5 ml; ammonium acetate, 5.5 g/I; CaC0₃, 5 g/I; pH adjusted to 6.5 with KOH; and autoclaved at 121°C for 20 minutes) are then added to solution A to prepare the aqueous culture medium.

### C. Preparation of Penicillium protoplasts

Cells from a 24 hour culture are harvested by suction filtration (Whatman #1 paper in a Buchner funnel) and suspended in buffer (0.01 M Tris; 0.01 M MgS0₄; 0.01 M DTT; 1.0 M KCI; and pH=7.0 with HCI). Sufficient buffer is added to obtain a final cell concentration of 1 g of cell mass per 50 ml of buffer. The cell suspension is placed on a gyratory water bath shaker in a 250 ml shake flask and incubated at 29-30°C for 10 minutes at 140 rpm with a 1 inch throw. Dithiothreitol-treated cells are collected by centrifugation and then resuspended in 50 ml of enzyme solution (10 mg/ml Novozym 234, Novo industri A/B Bagsvaerd, Denmark; 0.01 M Tris; 0.01 M MgS0₄; 0.01 M DTT; 1.0 M KCI; and pH-5.8 with HCI) in a 250 ml shake flask. This cell suspension is placed on a gyratory water-bath shaker and incubated at 29-30°C for 15 minutes at 140 rpm with a 1 inch throw. Enzyme-treated cells are centrifuged at 1240Xg for 6 min, and the resulting pellet is resuspended in buffer (0.01 M Tris-HCI; 0.01 M MgS0₄; 1.0 M KCl; and pH=7.0 with HCI). The suspension is first centrifuged at 950Xg for 6 minutes. The resulting pellet is resuspended in the same buffer, and the suspension is centrifuged at 700Xg for 6 minutes. The resulting pellet is resuspended in 5 ml of the same buffer. This suspension contains primarily large protoplasts and osmotically fragile cells that retain some cell wall structure. Compared to the small protoplasts removed by the above procedure, the percentage of protoplasts able to regenerate cell walls and percentage of viable osmotically stable cells is higher for the large protoplasts and osmotically fragile cells in the final suspension. The suspension of cells is diluted with buffer to a concentration of ~2 x 10⁸ cells/ml.

### D. Transformation Procedure

For each transforming plasmid, an ~0.1 ml suspension of osmotically fragile Penicillium chnysogenum cells (approximately 2 x 10⁷ cells) is supplemented with 10 µl of 50 mM CaCl₂, 25 µg of plasmid DNA in 5-15 µl of TE buffer, and 0.9 ml of a solution of freshly dissolved polyethylene glycol 4000 (Baker, 40% weight/volume in osmotically stabilized buffer). The mixture is vortexed, allowed to stand for 10 minutes at room temperature, centrifuged at 700Xg for 2 minutes, and vortexed again. Two aliquots of 0.5 ml each are then spread on the surface of osmotically stabilized acetamide medium (1.7 g/I Yeast Nitrogen Base without amino acids and ammonium sulfate; 125 g/I sucrose, 0.738 g/I acetamide; 1.27 g/I CaCl₂; and 22 g/I Noble agar). To measure the total number of viable cells present in transformation mixtures, aliquots from the transformation mixture are plated on medium in which the acetamide is replaced with an equimolar amount of ammonium sulfate. Seven to ten days after transformation, transformant colonies of sufficient size to subculture are present on the acetamide medium. Abortive transformants are easily distinguished from stable transformants, because abortive transformants fail to grow upon subculture to fresh acetamide medium. Cells transformed with a plasmid containing the acetamidase gene form visible colonies in four to five days after transformation.

### E. Analysis of Penicillium Transformants

Penicillium transformants that are transformed with vectors of the invention containing the amdS gene express an acetamidase activity (the amdS gene product) not detected in extracts of the untransformed recipient P. chrysogenum strain (e.g., ATCC 9480). This activity results in the ability of the transformed strains to grow using the ammonia released by acetamide hydrolysis when no other nitrogen sources are available. The Penicillium transformants of the invention also express epimerase activity

Stable transformants carry the transforming DNA in their high molecular weight DNA. Probes, e.g., the epimerase coding sequence or fragments of Aspergillus DNA that contain the acetamidase gene, hybridize to the high molecular weight DNA from these transformants even after multiple passage on non-selective medium (ammonia as nitrogen source). The transforming phenotype (production of epimerase and, if an amdS vector was used, ability to grow on acetamide as sole nitrogen source) is also maintained by the transformants after passage on non-selective medium.

## Claims (Claims for the following Contracting State(s) : AT, BE, CH, DE, DK, FR, GB, IT, LI, NL, SE)

1. An isolated DNA compound that comprises a DNA sequence encoding a Streptomyces lipmanii protein of amino acid sequence: 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGlnValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg
AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
 wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

2. The isolated DNA compound of Claim 1, wherein the coding strand comprises the DNA sequence: 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 wherein A is a deoxyadenyl residue, G is a deoxyguanyl residue, C is a deoxycytidyl residue, and T is a thymidyl residue.

3. A recombinant DNA vector that comprises the DNA sequence of Claim 1.

4. A recombinant DNA vector of Claim 3 that further comprises a promoter positioned to drive expression of said epimerase activity-encoding DNA.

5. The recombinant DNA expression vector of Claim 4, wherein said promoter functions in E. coli, Asperqillus, Penicillium, Cephalosporium, or Streptomyces.

6. The recombinant DNA expression vector of Claim 5, wherein said promoter is the λpL promoter or the promoter of the Aspergillus nidulans amdS gene.

7. The recombinant DNA expression vector of Claim 6 that is plasmid pOG0299 obtainable by subjecting mOG0292 (NRRL B-18511) to site-specific mutagenesis to create an NCOI restriction enzyme recognition site at the 5' end of the S. lipmanii epimerase coding sequence, resulting in plasmid mOG0297, and ligating the -3.8 kb BamHl-Ncol fragment from mOG0297, the~5.75 kb Xbal-BamHl vector fragment from pCZR111 (NRRL B-18249) and a double-stranded Xbal-Ncol restriction fragment synthesised by the phosphotriester method.

8. The recombinant DNA vector of Claim 3 that is mOG0292 obtainable from NRRL B-18511.

9. A method for constructing a recombinant host cell capable of expressing epimerase activity, said method comprising:
transforming said host cell with a recombinant DNA expression vector that comprises:
(a) a promoter and translational activating sequence that function in said host cell; and
(b) a DNA sequence encoding epimerase activity of Streptomyces lipmanii positioned for expression from said promoter and translational activating sequence.

10. A recombinant DNA vector selected from the group consisting of
mOG0292 obtainable from NRRL B-18511;
mOG0297 obtainable by subjecting mOG0292 to site-specific mutagenis to create an Ncol restriction enzyme recognition site at the 5' end of the S lipmanii epimerase coding sequence, and pOG0299 obtainable by subjecting mOG0292 (NRRL B-18511) to site-specific mutagenesis subjecting mOG0292 to site-specific mutagenis to create an NcOI restriction enzyme recognition site at the 5' end of the S. lipmanii epimerase coding sequence, resulting in plasmid mOG0297, and ligating the ~3.8 kb BamHI-Ncol fragment from mOG0297, the~5.75 kb Xbal-BamHl vector fragment from pCZR111 (NRRL B-18249) and a double-stranded Xbal-Ncol restriction fragment synthesised by the phosphotriester method.

## Claims (Claims for the following Contracting State(s) : ES, GR)

1. A method for constructing a recombinant host cell capable of expressing epimerase activity, said method comprising:
transforming said host cell with a recombinant DNA expression vector that comprises:
(a) a promoter and translational activating sequence that function in said host cell; and
(b) a DNA sequence encoding a Streptomyces lipmanni protein of amino acid sequence: 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGlnValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg
AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, lle is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue;
positioned for expression from said promoter and translational activating sequence.

2. A method according to Claim 1 in which the DNA sequence comprises: 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 wherein A is a deoxyadenyl residue, G is a deoxyguanyl residue, C is a deoxycytidyl residue, and T is a thymidyl residue.

## Patentansprüche (Patentansprüche für folgende(n) Vertragsstaat(en) : AT, BE, CH, DE, DK, FR, GB, IT, LI, NL, SE)

1. Isolierte DNA Verbindung, die eine DNA Sequenz enthält, welche ein Streptomyces lipmanii Protein mit der folgenden Ammosäuresequenz kodiert: 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGlnValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg
AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
 worin Ala für einen Alaninrest steht, Arg für einen Argininrest steht, Asn für einen Asparaginrest steht, Asp für einen Asparaginsäurerest steht, Cys für einen Cysteinrest steht, Gln für einen Glutaininrest steht, Glu für einen Glutaminsäurerest steht, Gly für einen Glycinrest steht, His für einen Histidinrest steht, Ile für einen Isoleucinrest steht, Leu für einen Leucinrest steht, Lys für einen Lysinrest steht, Met für einen Methioninrest steht, Phe für einen Phenylalaninrest steht, Pro für einen Prolinrest steht, Ser für einen Serinrest steht, Thr für einen Threoninrest steht, Trp für einen Tryptophanrest steht, Tyr für einen Tyrosinrest steht und Val für einen Valinrest steht.

2. Isolierte DNA Verbindung nach Anspruch 1, worin der kodierende Strang die folgende DNA Sequenz enthält 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 worin A ein Desoxyadenylrest ist, G ein Desoxyguanylrest ist, C ein Desoxycytidylrest ist und T ein Thymidylrest ist.

3. Rekombinanter DNA Vektor, der die DNA Sequenz nach Anspruch 1 enthält.

4. Rekombinanter DNA Vektor nach Anspruch 3, der ferner einen Promotor enthält, der zur Expressionssteuerung dieser Epüneraseaküvhät-kodierenden DNA positioniert ist.

5. Rekombinanter DNA Expressionsvektor nach Anspruch 4, worin der Promotor in E. coli, As-pegillus, Penicillium, Cephalosporium oder Streptomyces funktionsfähig ist.

6. Rekombinanter DNA Expressionsvektor nach Anspruch 5, worin dieser Promotor der λpL Promotor oder der Promotor des Asperqillus nidulans amdS Gens ist.

7. Rekombinanter DNA Expressionsvektor nach Anspruch 6, der das Plasmid pOG0299 ist, das erhalten wird, indem man mOG0292 (NRRL B-18511) einer ortsspezifischen Mutagenese unter Bildung einer Ncol Restriktionsschnittstelle am 5'-Ende der für die Epimerase von S. lipmanii kodierenden Sequenz unterzieht, was zu Plasmid mOG0297 führt und das -3,8 kb BamHl-Ncol Fragment von mOG0297, das -5,75 kb Xbal-BamHI Vektorfragment von pCZR111 (NRRL B-18249) und ein doppelsträngiges Xbal-Ncol Restriktionsfragment, das durch das Phosphotriesterverfahren hergestellt wurde, ligiert.

8. Rekombinanter DNA Vektor nach Anspruch 3, der mOG0292 ist und von NRRL B-18511 erhalten werden kann.

9. Verfahren zur Konstruktion einer rekombinanten Wirtszelle, die zur Expression einer Epimeraseaktivität fähig ist, gekennzeichnet durch
Transformation dieser Wirtszelle mit einem rekombinanten DNA Expressionsvektor, der enthält:
(a) Einen Promotor und eine Translationsaktivierungssequenz, die in dieser Wirtszelle funktionsfähig sind, und
(b) eine DNA Sequenz, die die Epimeraseaktivität von Streptomyces lipmanii kodiert und zur Expression von diesem Promotor und dieser Translationsaktivierungssequenz positioniert ist.

10. Rekombinanter DNA Vektor, der aus der Gruppe ausgewählt ist, die besteht aus
mOG0292, erhältlich von NRRL B-18511
mOG0297, erhältlich, indem man mOG0292 einer ortsspezifischen Mutagenese unter Bildung einer Ncol Restriktionsschnittstelle am 5'-Ende der für Epimerase kodierenden Sequenz von S. lipmanii unterzieht, und pOG0299, erhältlich, indem man mOG0292 (NRRL B-18511) einer ortsspezifischen Mutagenese unter Bildung einer Ncol Restriktionsschnittstelle am 5'-Ende der für die Epimerase von S.lipmanii kodierenden Sequenz unterzieht, was zu Plasmid mOG0297 führt, und das ~3,8 kb BamHI-Ncol Fragment von mOG0297, das -5,75 kb Xbal-BamHl Vektorfragment von pCZR111 (NRRL B-18249) und ein doppelsträngiges Xbal-Ncol Restriktions-fragment, das durch das Phosphotriesterverfahren synthetisiert wurde, ligiert.

## Patentansprüche (Patentansprüche für folgende(n) Vertragsstaat(en) : ES, GR)

1. Verfahren zur Konstruktion einer rekombinanten Wirtszelle, die zur Expression einer Epimeraseaktivität fähig ist, gekennzeichnet durch
Transformation dieser Wirtszelle mit einem rekombinanten DNA Expressionsvektor, der enthält:
(a) Einen Promotor und eine Translationsaktivierungssequenz, die in dieser Wirtszelle funktionsfähig sind, und
(b) eine DNA Sequenz, die ein Protein von Streptomyces lipmanii mit folgender Aminosäuresequenz kodiert: 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGlnValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg
AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
 worin Ala für einen Alaninrest steht, Arg für einen Argininrest steht, Asn für einen Asparaginrest steht, Asp für einen Asparaginsäurerest steht, Cys für einen Cysteinrest steht, Gin für einen Glutaminrest steht, Glu für einen Glutaminüsäurerest steht, Gly für einen Glycinrest steht, His für einen Histidinrest steht, Ile für einen Isoleucinrest steht, Leu für einen Leucinrest steht, Lys für einen Lysinrest steht, Met für einen Methioninrest steht, Phe für einen Phenylalaninrest steht, Pro für einen Prolinrest steht, Ser für einen Serinrest steht, Thr für einen Threoninrest steht, Trpfür einen Tryptophanrest steht, Tyrfür einen Tyrosinrest steht und Val für einen Valinrest steht,
und die zur Expression von diesem Promotor und dieser Translationsaktivierungssequenz positioniert ist.

2. Verfahren nach Anspruch 1, worin die DNA Sequenz enthält 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 worin A ein Desoxyadenylrest ist, G ein Desoxyguanylrest ist, C ein Desoxycytidylrest ist und T ein Thymidylrest ist.

## Revendications (Revendications pour l'(les) Etat(s) contractant(s) suivant(s) : AT, BE, CH, DE, DK, FR, GB, IT, LI, NL, SE)

1. Composé d'ADN isolé qui comprend une séquence d'ADN encodant une protéine de Streptomyces lipmanii présentant la séquence d'acides aminés : 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGluValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg
AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
 dans laquelle Ala représente un résidu d'alanine, Arg représente un résidu d'arginine, Asn représente un résidu d'asparagine, Asp représente un résidu d'acide aspartique, Cys représente un résidu de cystéine, Gln représente un résidu de glutamine, Glu représente un résidu d'acide glutamique, Gly représente un résidu de glycine, His représente un résidu d'histidine, Ile représente un résidu d'isoleucine, Leu représente un résidu de leucine, Lys représente un résidu de lysine, Met représente un résidu de méthionine, Phe représente un résidu de phénylalanine, Pro représente un résidu de proline, Ser représente un résidu de sérine, Thr représente un résidu de thréonine, Trp représente un résidu de tryptophane, Tyr représente un résidu de tyrosine et Val représente un résidu de valine.

2. Composé d'ADN isolé selon la revendication 1, dans lequel le brin codant comprend la séquence d'ADN: 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 dans laquelle A représente un résidu désoxyadényle, G représente un résidu désoxyguanyle, C représente un résidu désoxycytidyle et T représente un résidu thymidyle.

3. Vecteur d'ADN recombinant qui comprend la séquence d'ADN selon la revendication 1.

4. Vecteur d'ADN recombinant selon la revendication 3, qui comprend en outre un promoteur positionné de façon à induire l'expression dudit ADN encodant une activité d'épimérase

5. Vecteur d'expression d'ADN recombinant selon la revendication 4, dans lequel ledit promoteur fonctionne dans_E. coli, Asperqillus, Penicillium, Cephalosporium ou Streptomyces.

6. Vecteur d'expression d'ADN recombinant selon la revendication 5, dans lequel ledit promoteur est le promoteur λpL ou le promoteur du gène amdS d'Aspergillus nidulans.

7. Vecteur d'expression d'ADN recombinant selon la revendication 6, qui est le plasmide pOG0299 pouvant être obtenu en soumettant mOG0292 (NRRL B-18511) à une mutagenèse spécifique aux sites de façon à créer un site de reconnaissance d'enzyme de restriction Ncol à l'extrémité 5' de la séquence codante de l'épimérase de S. lipmanii, donnant le plasmide mOG0297, et en liant le fragment BamHi-Ncol d'environ 3,8 kb de mOG0297, le fragment de vecteur Xbal-BamHl d'environ 5,75kb de pCZR111 (NRRL B-18249) et un fragment de restriction double-brin Xbal-Ncol synthétisé par la méthode du phosphotriester

8. Vecteur d'ADN recombinant selon la revendication 3, qui est mOG0292 pouvant être obtenu à partir de NRRL B-18511.

9. Méthode de construction d'une cellule-hôte recombinante capable d'exprimer une activité d'épimérase, ladite méthode comprenant : la transformation de ladite cellule-hôte avec un vecteur d'expression d'ADN recombinant qui comprend :
(a) un promoteur et une séquence d'activation de la traduction qui fonctionnent dans ladite cellule-hôte; et
(b) une séquence d'ADN codant pour une activité d'épimérase de Streptomyces lipmanii, positionnée pour l'expression à partir dudit promoteur et de ladite séquence d'activation de la traduction.

10. Vecteur d'ADN recombinant choisi parmi le groupe constitué de mOG0292 pouvant être obtenu à partir de NRRL B-18511;
mOG0297 pouvant être obtenu en soumettant mOG0292 à une mutagenèse spécifique aux sites de façon à créer un site de reconnaissance d'enzyme de restriction Ncol à l'extrémité 5' de la séquence codante de l'épimérase de S. lipmanii, et
pOG0299 pouvant être obtenu en soumettant mOG0292 (NRRL B-18511) à une mutagenèse spécifique aux sites de façon à créer un site de reconnaissance d'enzyme de restriction Ncol à l'extrémité 5' de la séquence codante de l'épimérase de S. lipmanii donnant le plasmide mOG0297, et en liant le fragment BamHI-Ncol d'environ 3,8 kb de mOG0297, le fragment de vecteur Xbal-BamHl d'environ 5,75 kb de pCZR111 (NRRL B-18249) et un fragment de restriction double-brin Xbal-Ncol synthétisé par la méthode du phosphotriester.

## Revendications (Revendications pour l'(les) Etat(s) contractant(s) suivant(s) : ES, GR)

1. Méthode de construction d'une cellule-hôte recombinante capable d'exprimer une activité d'épimérase, ladite méthode comprenant: la transformation de ladite cellule-hôte avec un vecteur d'expression d'ADN recombinant qui comprend : (a) un promoteur et une séquence d'activation de la traduction qui fonctionnent dans ladite cellule-hôte; et (b) une séquence d'ADN encodant une protéine de Streptomyces lipmanii présentant la séquence d'acides aminés : 
MetAlaCysSerAspTrpGlyAspAlaArgArgArgMetLeuLeuAspProThrAlaVal
AsnLeuAsnThrGlySerAlaGlyProLeuProArgThrValPheAspHisValThrGly
LeuArgAlaArgLeuAlaHisAlaProMetAspPheLeuLeuArgGluValProGlyLeu
LeuTrpGlnAlaArgGluSerLeuAlaGlyPheValGlyGlyAspProHisArgLeuAla
PheThrThrAsnValThrGlyAlaValAsnLeuValAlaThrSerLeuProProLeuAla
ProGlyGluIleLeuMetThrAspHisGluTyrProProMetArgTrpCysTrpGluArg
ThrAlaArgArgGlnGlyLeuSerValArgThrPheProLeuProGluMetProGluSer
ProAlaGluIleValAspAlaValValAlaAlaLeuGlyProArgThrArgValLeuPhe
LeuSerHisValLeuSerAlaThrGlyLeuIleLeuProValArgGluLeuCysGluGlu
AlaArgArgArgGlyIleValThrValIleAspGlyAlaHisAlaProGlyTyrValAsp
LeuAspLeuThrArgIleProCysAspPheTyrValGlySerGlyHisLysTrpLeuLeu
AlaProThrGlyValGlyPheLeuHisIleGlyProGlyArgAlaAspAspLeuGluPro
ThrGlnValSerTrpAlaTyrGluProProProGlySerAspProAspArgArgAspArg
PheGlySerThrProArgLeuArgArgLeuGluCysGluGlyThrArgAspLeuCysPro
TrpLeuThrValProGluThrIleArgPheGlnAlaGlyLeuGlyHisAspArgIleArg
AlaArgArgArgGluLeuAlaAspHisAlaArgArgArgLeuThrAspGlyGlnGlyLeu
LysProAlaThrProGluProSerSerLeuSerGlyGlyMetThrAlaPheHisLeuPro
AlaThrValArgAlaAspGluValArgGlnGlyLeuTrpGluArgPheArgIleGluThr
AlaValSerAspGlyProAsnGlyProLeuLeuArgIleSerAlaAsnPheTyrThrThr
GluGluGluIleAspHisLeuAlaAspSerLeuLysAspLeuLeuAlaGly
 dans laquelle Ala représente un résidu d'alanine, Arg représente un résidu d'arginine, Asn représente un resiau d'asparagine, Asp représente un résidu d'acide aspartique, Cys représente un résidu de cystéine, Gln représente un résidu de glutamine, Glu représente un résidu d'acide glutamique, Gly représente un résidu de glycine, His représente un résidu d'histidine, Ile représente un résidu d'isoleucine, Leu représente un résidu de leucine, Lys représente un résidu de lysine, Met représente un résidu de méthionine, Phe représente un résidu de phénylalanine, Pro représente un résidu de proline, Ser représente un résidu de sérine, Thr représente un résidu de thréonine, Trp représente un résidu de tryptophane, Tyr représente un résidu de tyrosine et Val représente un résidu de valine;
positionné pour l'expression à partir dudit promoteur et de ladite séquence d'activation de la traduction.

2. Méthode selon la revendication 1, dans laquelle la séquence d'ADN comprend 
1 ATGGCCTGTT CCGACTGGGG CGATGCCCGT CGCCGCATGC TGCTCGACCC
51 CACCGCCGTC AACCTCAACA CCGGCTCCGC GGGCCCGCTC CCGCGCACCG
101 TCTTCGACCA CGTCACCGGG TTGCGCGCCC GCCTCGCCCA CGCGCCGATG
151 GACTTCCTGC TGCGCGAGGT CCCCGGGCTG CTGTGGCAGG CGCGCGAGAG
201 CCTCGCCGGC TTCGTGGGAG GCGACCCGCA CCGGCTCGCG TTCACCACCA
251 ACGTCACCGG GGCCGTCAAC CTGGTGGCGA CATCGCTGCC CCCGCTCGCC
301 CCGGGCGAGA TCCTGATGAC CGACCACGAG TACCCGCCGA TGCGCTGGTG
351 CTGGGAGCGC ACGGCCCGAC GGCAGGGCCT GTCGGTCAGG ACGTTCCCGC
401 TGCCGGAGAT GCCCGAGAGC CCCGCGGAGA TCGTCGACGC CGTCGTCGCC
451 GCGCTTGGAC CGCGGACCCG AGTCCTCTTC CTCAGCCACG TACTGTCGGC
501 GACCGGCCTG ATCCTGCCGG TCCGGGAACT GTGCGAGGAG GCCCGCCGGC
551 GCGGGATCGT CACGGTGATC GACGGCGCCC ACGCGCCCGG GTACGTCGAC
601 CTGGACCTGA CCCGCATCCC GTGCGACTTC TACGTGGGCA GCGGGCACAA
651 GTGGCTCCTC GCCCCCACCG GGGTCGGCTT CCTCCACATC GGCCCGGGAC
701 GGGCGGACGA CCTGGAGCCG ACACAGGTGA GCTGGGCCTA CGAACCGCCG
751 CCGGGAAGCG ATCCGGACCG GCGCGACCGG TTCGGCAGCA CCCCCCGGCT
801 GCGCCGGCTG GAATGCGAGG GCACCCGCGA CCTGTGCCCC TGGCTGACCG
851 TGCCGGAGAC GATCCGCTTC CAGGCCGGAC TGGGCCACGA CAGGATCCGC
901 GCCCGCAGGC GCGAGCTGGC CGATCACGCC CGCCGGCGCC TGACCGACGG
951 GCAGGGGCTG AAACCGGCCA CTCCTGAACC GTCATCCCTG TCCGGCGGAA
1001 TGACGGCATT TCACCTGCCC GCGACCGTAC GAGCGGACGA GGTGCGTCAG
1051 GGCCTCTGGG AACGTTTCCG GATCGAGACC GCCGTGTCCG ACGGACCGAA
1101 CGGGCCACTC CTGCGAATAT CGGCGAATTT CTACACCACC GAAGAGGAAA
1151 TCGATCACCT GGCGGACTCA CTGAAGGATC TCCTGGCGGG CTGA
 dans laquelle A représente un résidu désoxyadényle, G représente un résidu désoxyguanyle, C représente un résidu désoxycytidyle et T représente un résidu thymidyle.
